# FASCICULE DE BREVET EUROPEEN

(11) **EP 1 129 684 B1**
(45) Date de publication et mention de la délivrance du brevet: **21.06.2006**
(21) Numéro de dépôt: 00403527.5
(22) Date de dépôt: 14.12.2000
(51) Int. Cl.: A61K 8/06, A61K 8/41, A61Q 99/00

(54) **Nanoémulsion à base de lipides amphiphiles et de polymères cationiques et utilisation en cosmétologie**
Aus amphiphilen Fettstoffen und ein kationisches Polymer bestehende Nanoemulsionen und deren Verwendungen in Kosmetica
Nanoemulsion based on amphiphilic lipids and cationic polymers and its use in cosmetics

(30) Priorité: 21.01.2000 FR 0000792
(43) Date de publication de la demande: 05.09.2001
(73) Titulaire: L'ORÉAL, 75008 Paris (FR)
(72) Inventeur: Douin, Véronique, 75017 Paris (FR); Cazin, Bénédicte, 92110 Clichy (FR); Decoster, Sandrine, 95210 Saint Gratien (FR)
(74) Mandataire: Le Blainvaux Bellegarde, Françoise

(56) Documents cités:
- EP-A- 0 270 249
- WO-A-99/53889

## Description

La présente invention a trait à une nanoémulsion huile-dans-eau dont les globules d'huile ont une taille moyenne inférieure à 150 nm et comprenant au moins un lipide amphiphile de préférence non ionique ou anionique et au moins un polymère cationique comportant au moins une séquence hydrophobe et au moins une séquence hydrophile ainsi qu'à leur utilisation en application topique notamment en cosmétique et en dermopharmacie.

Les émulsions huile-dans-eau sont bien connues dans le domaine de la cosmétique et de la dermopharmacie notamment pour la préparation de produits cosmétiques tels que des lotions, des toniques, des sérums, des eaux de toilette.

Cependant, la présence de concentrations importantes d'huiles végétales, animales ou minérales dans des compositions rend leur formulation difficiles. En effet, les compositions sont généralement instables au stockage et les propriétés cosmétiques sont insuffisantes. En particulier, l'application de telles compositions sur les cheveux entraîne un toucher gras, une difficulté de rinçage. De plus, les cheveux séchés sont sans volume et ont un toucher chargé.

Par nanoémulsion, on entend une émulsion H/E métastable dont la taille des globules huileux est inférieure à 150 nm, globules huileux stabilisés par une couronne de lipides amphiphiles pouvant éventuellement former une phase cristal liquide de type lamellaire situés à l'interface huile/phase aqueuse. La transparence de ces émulsions provient de la petite taille des globules huileux, petite taille obtenue grâce à l'utilisation notamment d'un homogénéisateur haute pression. Les nanoémulsions sont à différencier des microémulsions de par leur structure. Les microémulsions sont des dispersions thermodynamiquement stables constituées de micelles de lipide (s) amphiphile (s) gonflées par de l'huile. De plus les microémulsions ne nécessitent pas d'énergie mécanique importante pour être réalisée. Elles se forment spontanément par simple mise en contact des constituants. Les inconvénients majeurs des microémulsions sont liés à leur forte proportion en tensioactifs, conduisant à des intolérances et entraînant un toucher collant lors de l'application sur la peau. Par ailleurs, leur domaine de formulation est en général très étroit et leur stabilité en température très limitée.

La phase lipidique amphiphile est composée d'un ou plusieurs (mélange) lipide amphiphile non ionique et/ou ionique. Par lipide amphiphile, on entend toutes molécules ayant une structure bipolaire comportant au moins une partie hydrophobe et au moins une partie hydrophile ayant la propriété de réduire la tension superficielle de l'eau (γ< 55mN/m) et de réduire la tension interfaciale entre l'eau et une phase huileuse. Les synonymes de lipide amphiphile sont par exemple: tensio-actif, agent de surface, emulsionnant.

On connaît dans l'état de la technique des nanoémulsions comprenant une phase lipidique amphiphile constituée de phospholipides, d'un lipide cationique, d'eau et d'un filtre solaire hydrophobe. Elles sont obtenues par un procédé d'homogénéisation à haute pression. Ces émulsions présentent l'inconvénient d'être instables au stockage aux températures traditionnelles de conservation à savoir entre 0 et 45°C. Elles conduisent à des compositions jaunes et produisent des odeurs de rance qui se développent après quelques jours de conservation. De plus, ces émulsions n'apportent pas de bonnes propriétés cosmétiques. Elles sont décrites dans la revue « DCI » d'avril 1996, pages 46-48.

Par ailleurs, les documents EP-A-728 460 et EP-A-780 114 décrivent des nanoémulsions à base de lipides amphiphiles non-ioniques fluides ou de tensioactifs siliconés.

Cependant toutes ces nanoémulsions sont fluides. Pour certaines utilisations on recherche des produits que l'on peut doser et prendre aisément dans la main. Pour ce faire, ces produits doivent présenter une certaine consistance ou viscosité. En effet, un produit liquide est beaucoup plus difficile à doser et s'écoule facilement entre les doigts.

Il est connu d'utiliser en tant qu'agent épaississant des milieux aqueux, des polymères hydrosolubles ou hydrodispersibles, et notamment des polymères éventuellement réticulés tels que les acides polycarboxy vinyliques et notamment les carbopol, lesdits polymères ayant de préférence une longueur de chaîne importante et un poids moléculaire élevé.

Malheureusement, ceux-ci ne permettent pas d'obtenir des compositions sous forme de nanoémulsions stables et transparentes.

Ainsi, il subsiste le besoin d'un système épaississant permettant d'épaissir, voire de gélifier, de manière convenable une composition sous forme de nanoémulsion huile-dans-eau, sans influer sur les propriétés cosmétiques desdites compositions.

La demanderesse a découvert, de façon inattendue, que l'on pouvait épaissir les nanoémulsions comportant une phase huileuse dispersée dans une phase aqueuse dont les globules d'huile ont une taille moyenne inférieure à 150 nm, avec des polymères cationiques de préférence hydrosolubles ou hydrodispersibles comportant au moins une séquence hydrophobe et au moins une séquence hydrophile.

La présente invention a pour objet des nanoémulsions huile-dans-eau comportant une phase huileuse dispersée dans une phase aqueuse dont les globules d'huile ont une taille moyenne en nombre inférieure à 150 nm, caractérisée par le fait qu'elles comprennent au moins une huile, au moins un lipide amphiphile , de préférence non ionique ou anionique et au moins un polymère cationique, de préférence hydrosoluble ou hydrodispersible, comportant au moins une séquence hydrophobe et au moins une séquence hydrophile et que le rapport pondéral de la quantité d'huile sur la quantité de lipide amphiphile est compris entre 1 et 10 et de préférence entre 1,2 et 6.

Un autre objet de l'invention est un procédé pour épaissir les nanoémulsions huile-dans-eau ayant des globules d'huile dont la taille moyenne en nombre est inférieure à 150 nm, dans lequel on ajoute à ladite composition un polymère cationique, de préférence hydrosoluble ou hydrodispersible, comportant au moins une séquence hydrophobe et au moins une séquence hydrophile.

Sans être tenu par cette explication, on peut considérer que, dans le cadre de l'invention, l'augmentation de la viscosité du milieu résulte de la formation d'un réseau de particules d'huile, ledit réseau faisant intervenir des associations de type hydrophobe entre, d'une part, les groupes hydrophobes du polymère et d'autre part, les coeurs hydrophobes des particules d'huile. L'existence de liens labiles entre les particules entraine une augmentation de la viscosité du mélange.

Les nanoémulsions conformes à l'invention sont préparées à des températures entre 4 et 45°C et sont compatibles avec des actifs thermosensibles. Elles peuvent contenir des quantités importantes d'huile. Elles peuvent notamment contenir des quantités importantes de parfum et améliorer leur rémanence. Elles favorisent également la pénétration des actifs dans les couches superficielles de la peau et le dépôt d'actif sur les fibres kératiniques telles que les cheveux. Les cheveux traités avec ces nanoémulsions sont brillants sans avoir un toucher ou un aspect gras, ils se démêlent facilement, sont plus doux et plus nerveux.

La composition cosmétique, et notamment capillaire, obtenue, s'étale aisément, présente une meilleure préhension, ainsi qu'une bonne élimination au rinçage.

Les polymères cationiques, de préférence hydrosolubles ou hydrodispersibles, comportent au moins une séquence hydrophobe et au moins une séquence hydrophile. De préférence, le polymère cationique comporte au moins deux séquences hydrophobes.

La ou les séquences hydrophobes sont principalement des chaînes grasses ayant de 6 à 30 atomes de carbone, notamment hydrocarbonées telles que alkyle, arylalkyle, alkylaryle, alcényle, des groupements divalents aliphatiques tels que alkylène en C4-C30, des groupements divalents cycloaliphatiques tels que notamment méthylènedicyclohexyl, isophorone ou des groupements divalents aromatiques tels que phénylène.

La ou les séquences hydrophiles, peuvent être, entre autre, un polyoxyde d'éthylène, un polysaccharide, un polyamide notamment polyacrylamide, un polyester et leurs mélanges et de préférence un polyoxyde d'éthylène ayant de 15 à 500 oxydes d'éthylène.

Le polymère cationique comprend au moins un groupement amine tertiaire ou quaternaire.

La ou les liaisons entre séquence hydrophobe et hydrophile est le plus souvent, sans être limitative, de type ester, éther, urée, amide ou uréthane et leurs mélanges.

Le rapport (en poids) de la (ou les) séquence(s) hydrophile(s) sur la (ou les) séquence(s) hydrophobe(s) du polymère, est de préférence compris entre 10/1 et 1000/1.

Les polymères cationiques utilisés dans la présente invention sont choisis de préférence parmi les dérivés de cellulose quaternisée et les polyacrylates à groupements latéraux aminés.
Les dérivés de cellulose quaternisée sont, en particulier,
- les celluloses quaternisées modifiées par des groupements comportant au moins une chaîne grasse, tels que les groupes alkyle, arylalkyle, alkylaryle, alcényle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle, alcényle comportant au moins 8 atomes de carbone, ou des mélanges de ceux-ci.
Les polyacrylates à groupements latéraux aminés, quaternisés ou non, de préférence non cycliques possèdent par exemple des groupements hydrophobes du type stéareth 20 (alcool stéarylique polyoxyéthyléné(20)) ou alkyl(C10-C30)PEG-20 itaconate.
Les radicaux alkyle portés par les celluloses ou hydroxyéthylcelluloses quaternisées ci-dessus comportent de préférence de 8 à 30 atomes de carbone.
Les radicaux aryle désignent de préférence les groupements phényle, benzyle, naphtyle ou anthryle.
On peut indiquer comme exemples d'alkylhydroxyéthyl-celluloses quaternisées à chaînes grasses en C₈₋₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C₁₈) commercialisés par la société CRODA;
Comme exemples de polyacrylates à chaînes latérales aminées non cycliques, on peut citer les polymères 8781-124B ou 9492-103 ou STRUCTURE PLUS de la société NATIONAL STARCH.

Selon l'invention, le ou les polymères cationiques comportant au moins une séquence hydrophobe et au moins une séquence hydrophile peuvent représenter de 0,1 % à 20 % en poids, de préférence de 0,5 % à 10% en poids et plus particulièrement de 1 à 5% en poids par rapport au poids total de la composition finale.

Les nanoémulsions selon la présente invention comprennent de préférence au moins un lipide amphiphile non ionique et/ou au moins un lipide amphiphile anionique.

Les lipides amphiphiles non-ioniques de l'invention sont préférentiellement choisis parmi :
1/- les tensioactifs siliconés,
2/- les lipides amphiphiles fluides à température inférieure ou égale à 45°C choisis parmi les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée,
3/- les esters mixtes d'acide gras ou d'alcool gras, d'acide carboxylique et de glycérol
4/- les esters d'acide gras et de sucre et les éthers d'alcool gras et de sucre
5/- les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés
6/- Les copolymères blocs d'oxyde d'éthylène (A) et d'oxyde de propylène (B),

1/ Les tensioactifs siliconés utilisables selon l'invention sont des composés siliconés comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5364633 et US-A-5411744.

De préférence, le tensioactif siliconé utilisé selon la présente invention est un composé de formule (I): 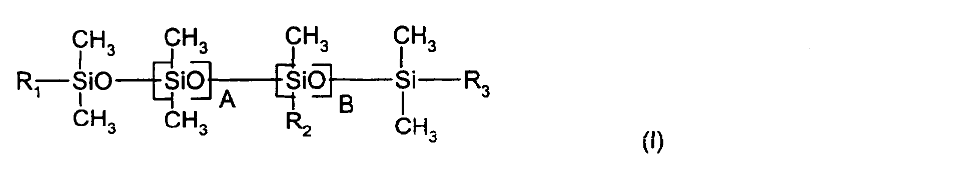 dans laquelle :
R₁, R₂, R₃, indépendamment les uns des autres, représentent un radical alkyle en C₁-C₆ ou un radical -(CH2)x - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200;
B est un nombre entier allant de 0 à 50 ; à la condition que A et B ne soient pas égaux à zéro en même temps ;
x est un nombre entier allant de 1 à 6;
y est un nombre entier allant de 1 à 30;
z est un nombre entier allant de 0 à 5.

Selon un mode de réalisation préféré de l'invention, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 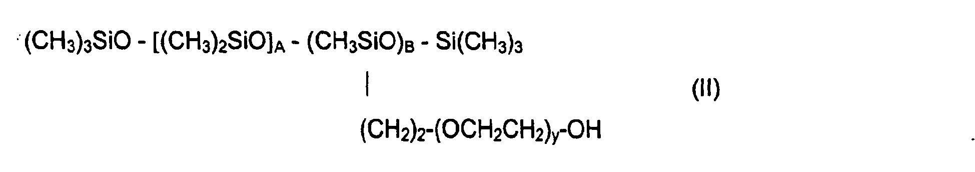 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (1), les composés de formule (III):

H - (OCH₂CH₂)_{y}-(CH₂)₃ - [(CH₃)₂SiO]_{A'} - (CH₂)₃ - (OCH₂CH₂)_{y} - OH (III)

dans laquelle A' et y sont des nombres entiers allant de 10 à 20.

On peut utiliser comme composés de l'invention ceux commercialisés par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12; A est 27, B est 3 et y est 12.

Le composé Q4-3667 est un composé de formule (III) où A est 15 et y est 13.

2/ les lipides amphiphiles fluides à température inférieure ou égale à 45°C sont notamment :
- l'isostéarate de polyéthylèneglycol de poids moléculaire 400, vendu sous la dénomination PEG 400 par la société UNICHEMA;
- l'isostéarate de diglycéryle, vendu par la société SOLVAY;
- le laurate de glycérol comportant 2 unités de glycérol , vendu par la société SOLVAY;
- L'oléate de sorbitane, vendu sous la dénomination SPAN 80 par la société ICI;
- l'isostéarate de sorbitane, vendu sous la dénomination NIKKOL SI 10R par la société NIKKO ;
- le cocoate d'α-butylglucoside ou le caprate d'α-butylglucoside commercialisés par la société ULICE.

3/ Les esters mixtes d'acide gras ou d'alcool gras, d'acide carboxylique et de glycérol, utilisables comme tensioactifs dans la nanoémulsion selon l'invention peuvent être choisis notamment dans le groupe comprenant les esters mixtes d'acide gras ou d'alcool gras ayant une chaîne alkyle comportant de 8 à 22 atomes de carbone, et d'alpha-hydroxyacide et/ou d'acide succinique, avec la glycérine. L'alpha-hydroxyacide peut être par exemple l'acide citrique, l'acide lactique, l'acide glycolique, l'acide malique et leurs mélanges.

La chaîne alkyle des acides ou alcools gras dont dérivent les esters mixtes utilisables dans la nanoémulsion de l'invention peut être linéaire ou ramifiée, saturée ou non saturée. Il peut s'agir notamment de chaînes stéarate, isostéarate, linoléate, oléate, béhénate, arachidonate, palmitate, myristate, laurate, caprate, isostéaryle, stéaryle, linoléyle, oléyle, béhényle, myristyle, lauryle, capryle et leurs mélanges.

On peut citer, à titre d'exemple d'esters mixtes utilisables dans la nanoémulsion de l'invention, l'ester mixte de glycérine et du mélange d'acides citrique, lactique, linoléique et oléique (nom CTFA : Glyceryl citrate/lactate/linoteate/oleate) commercialisé par la société Hüls sous la dénomination Imwitor 375; l'ester mixte d'acide succinique et d'alcool isostéarylique avec la glycérine (nom CTFA : Isostéaryl diglycéryl succinate) commercialisé par la société Hüls sous la dénomination Imwitor 780 K ; l'ester mixte d'acide citrique et d'acide stéarique avec la glycérine (nom CTFA : Glyceryl stearate citrate) commercialisé par la société Hüls sous la dénomination Imwitor 370 ; l'ester mixte d'acide lactique et d'acide stéarique avec la glycérine (nom CTFA : Glyceryl stearate lactate) commercialisé par la société Danisco sous la dénomination Lactodan B30 ou Rylo LA30.

4/ Les esters d'acide gras et de sucre, utilisables comme tensioactifs dans la nanoémulsion selon l'invention sont de préférence solides à une température inférieure ou égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose.

Les acides gras en C₈-C₂₂ ou en C₁₄-C₂₂ formant le motif gras des esters utilisables dans la nanoémulsion de l'invention comportent une chaîne alkyle linéaire saturée ou non saturée, comportant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.

On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'o-hexadécanoyle-6-D-glucoside de méthyle et l'o-hexadécanoyle-6-D-maltoside.

Les éthers d'alcool gras et de sucre, utilisables comme tensioactifs dans la nanoémulsion selon l'invention sont solides à une température inférieure ou égale à 45°C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.

Les alcools gras en C₈-C₂₂ ou en C₁₄-C₂₂ formant le motif gras des éthers utilisables dans la nanoémulsion de l'invention comportent une chaîne alkyle linéaire saturée ou non saturée, comportant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle, et leurs mélanges tels que cétéaryle.

A titre d'exemple d'éthers d'alcool gras et de sucre, on peut citer les alkylpolyglucosides tels que le décyl glucoside et le lauryl glucoside commercialisés par exemple par la société Henkel sous les dénominations respectives Plantaren 2000 et Plantaren 1200, le cétostéaryl glucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidyl glucoside commercialisé sous la dénomination Montanov 202 par la société Seppic.

On utilise plus particulièrement comme tensioactif le monostéarate de sucrose, le distéarate de sucrose, le tristéarate de sucrose et leurs mélanges, le distéarate de méthyl glucose et de polyglycérol-3 et les alkylpolyglucosides.

5/ Les esters gras de glycérol, utilisables comme tensioactifs dans la nanoémulsion selon l'invention solides à une température inférieure ou égale à 45°C peuvent être choisis notamment dans le groupe comprenant les esters formés d'au moins un acide comportant une chaîne alkyle linéaire saturée, ayant de 16 à 22 atomes de carbone, et de 1 à 10 motifs glycérol. On peut utiliser un ou plusieurs de ces esters gras de glycérol dans la nanoémulsion de l'invention.

Ces esters peuvent être notamment choisis parmi les stéarates, béhénates, arachidates, palmitates et leurs mélanges. On utilise de préférence des stéarates et palmitates.

On peut citer à titre d'exemple de tensioactif utilisable dans la nanoémulsion de l'invention, les monostéarate, distéarate, tristéarate et pentastéarate de décaglycérol (noms CTFA : Polyglyceryl-10 stearate, Polyglyceryl-10 distearate, Polyglyceryl-10 tristearate, Polyglyceryl-10 pentastearate) tels que les produits vendus sous les dénominations respectives Nikkol Decaglyn 1-S, 2-S, 3-S et 5-S par la société Nikko, et le monostéarate de diglycérol (nom CTFA: Polyglyceryl-2 stearate) tel que le produit vendu par la société Nikko sous la dénomination Nikkol DGMS.

Les esters gras de sorbitan, utilisables comme tensioactifs dans la nanoémulsion selon l'invention sont solides à une température inférieure ou égale à 45°C et sont choisis dans le groupe comprenant les esters d'acide gras en C₁₆-C₂₂ et de sorbitan et les esters d'acide gras en C₁₆-C₂₂ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'éthylène glycol et de préférence de 2 à 40 unités d'oxyde d'éthylène (OE).

Ces esters peuvent être notamment choisis parmi les stéarates, béhénates, arachidates, palmitates, et leurs mélanges. On utilise de préférence des stéarates et palmitates.

On peut citer à titre d'exemple de tensioactif utilisable dans la nanoémulsion de l'invention, le monostéarate de sorbitan (nom CTFA: Sorbitan stearate) vendu par la société ICI sous la dénomination Span 60, le monopalmitate de sorbitan (nom CTFA: Sorbitan palmitate) vendu par la société ICI sous la dénomination Span 40, le tristéarate de sorbitan 20 OE (nom CTFA : Polysorbate 65) vendu par la société ICI sous la dénomination Tween 65.

Les éthers gras éthoxylés solides à une température inférieure ou égale à 45°C, utilisables comme tensioactifs dans la nanoémulsion selon l'invention sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société Nikko, et l'éther d'alcool stéarylique comprenant 2 unités d'oxyde d'éthylène (nom CTFA: Steareth-2), tel que le produit commercialisé sous la dénomination Brij 72 par la société ICI.

Les esters gras éthoxylés solides à une température inférieure ou égale à 45°C, utilisables comme tensioactifs dans la nanoémulsion selon l'invention sont des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA : PEG-8 behenate), tel que le produit commercialisé sous la dénomination Compritol HD5 ATO par la société Gattefosse.

6/ Les copolymères blocs d'oxyde d'éthylène (A) et d'oxyde de propylène (B), utilisables comme tensioactifs dans la nanoémulsion selon l'invention peuvent être choisis notamment parmi les copolymères blocs de formule (I):

HO(C₂H₄O)x(C₃H₆O)y(C₂H₄O)zH (I)

dans laquelle x, y et z sont des nombres entiers tels que x+z va de 2 à 100 et y va de 14 à 60, et leurs mélanges, et plus particulièrement parmi les copolymères blocs de formule (I) ayant un HLB allant de 2 à 16.

Ces copolymères blocs peuvent être notamment choisis parmi les poloxamers et notamment parmi le Poloxamer 231 tel que le produit commercialisé par la société ICI sous la dénomination Pluronic L81 de formule (I) avec x=z=6, y=39 (HLB 2) ; le Poloxamer 282 tel que le produit commercialisé par la société ICI sous la dénomination Pluronic L92 de formule (I) avec x=z=10, y=47 (HLB 6) ; et le Poloxamer 124 tel que le produit commercialisé par la société ICI sous la dénomination Pluronic L44 de formule (I) avec x=z=11, y=21 (HLB 16).

Parmi les lipides amphiphiles non ioniques, on préfère utiliser:
- l'isostéarate de polyéthylèneglycol (8 moles d'oxyde d'éthylène),
- l'isostéarate de diglycéryle,
- le monolaurate et le monostéarate de polyglycérol comportant 10 unités de glycérol ,
- l'oléate de sorbitane,
- l'isostéarate de sorbitane,

Les lipides amphiphiles anioniques de l'invention sont notamment choisis parmi :
- Les citrates d'alkyléther
- Les alkényl succinates alkoxylés
- Les alkényl succinates de glucose alkoxylés
- Les alkényl succinates de méthylglucose alkoxylés.

Les citrates d'alkyléther utilisables comme tensioactifs dans la nanoémulsion selon l'invention peuvent être choisis notamment dans le groupe comprenant les monoesters, diesters ou triesters formés par l'acide citrique et au moins un alcool gras oxyéthyléné, comportant une chaîne alkyle linéaire ou ramifiée, saturée ou non saturée, ayant de 8 à 22 atomes de carbone, et comportant de 3 à 9 groupes éthoxylés, et leurs mélanges. On peut en effet utiliser un mélange d'un ou plusieurs de ces citrates dans la nanoémulsion de l'invention.

Ces citrates peuvent être par exemple choisis parmi les mono-, di- et tri-esters d'acide citrique et d'alcool laurique éthoxylé, comportant de 3 à 9 groupes éthoxylés, commercialisés par la société Witco sous la dénomination Witconol EC, en particulier le Witconol EC 2129 qui est majoritairement un dilaureth-9 citrate, et le Witconol EC 3129 qui est majoritairement un trilaureth-9 citrate.

Les citrates d'alkyléther, utilisés comme tensioactifs sont de préférence employés sous forme neutralisée à un pH d'environ 7, l'agent de neutralisation étant choisi parmi les bases inorganiques telles que la soude, la potasse, l'ammoniac, et les bases organiques telles que la mono-, di- et tri-éthanolamine, l'aminométhylpropanediol-1,3, la N-méthylglucamine, les acides aminés basiques comme l'arginine et la lysine, et leurs mélanges.

Les alkényl succinates utilisables comme tensioactif dans la nanoémulsion de l'invention sont notamment des dérivés éthoxylés et/ou propoxylés et ils sont de préférence choisis parmi les composés de formules (I) ou (II) :

HOOC-(HR)C-CH₂-COO-E (I)

HOOC-(HR)C- CH₂-COO-E-O-CO- CH₂-C(HR')-COOH (II)

dans lesquelles :
- les radicaux R et R' sont choisis parmi les radicaux alkényle, linéaires ou ramifiés, comportant de 6 à 22 atomes de carbone,
- E est choisi parmi les chaînes oxyéthylénées de formule (C₂H₄O)ₙ dans laquelle n va de 2 à 100, les chaînes oxypropylénées de formule (C₃H₆O)ₙ dans laquelle n' va de 2 à 100, les copolymères statistiques ou séquencés comprenant des chaînes oxyéthylénées de formule (C₂H₄O)ₙ et des chaînes oxypropylénées de formule (C₃H₆O)ₙ, telles que la somme de n et n' va de 2 à 100, les groupements glucoses oxyéthylénés et/ou oxypropylénés comportant en moyenne de 4 à 100 motifs oxyéthylénés et/ou oxypropylénés répartis sur l'ensemble des fonctions hydroxyle, les groupements méthyl glucoses oxyéthylénés et/ou oxypropylénés comportant en moyenne de 4 à 100 motifs oxyéthylénés et/ou oxypropylénés répartis sur l'ensemble des fonctions hydroxyle.

Dans les formules (I) et (II), n et n' sont des valeurs moyennes et ne sont donc pas forcément des entiers. On choisit avantageusement pour n une valeur allant de 5 à 60 et encore plus préférentiellement de 10 à 30.

Avantageusement, le radical R et/ou R' est choisi parmi les radicaux alkényle linéaires comportant de 8 à 22 et de préférence de 14 à 22 atomes de carbone. Il peut s'agir par exemple du radical hexadécényl comportant 16 atomes de carbone ou du radical octadécényl comportant 18 atomes de carbone.

Les composés de formules (I) et (II) décrits ci-dessus dans lesquels E est choisi parmi les chaînes oxyéthylénées, les chaînes oxypropylénées et les copolymères comprenant des chaînes oxyéthylénées et des chaînes oxypropylénées, peuvent être préparés conformément à la description qui est donnée dans les documents WO-A-94/00508, EP-A-107199 et GB-A-2131820 incorporés ici pour référence.

La fonction acide -COOH des tensioactifs de formules (I) et (II) se trouve en général dans la nanoémulsion de l'invention, sous forme neutralisée par un agent de neutralisation, l'agent de neutralisation étant choisi par exemple parmi les bases inorganiques telles que la soude, la potasse, l'ammoniac, et les bases organiques telles que la mono-, di- et tri-éthanolamine, l'aminométhylpropanediol-1,3, la N-méthyl-glucamine, les acides aminés basiques comme l'arginine et la lysine, et leurs mélanges.

A titre d'exemple de tensioactif utilisable dans la nanoémulsion de l'invention, on peut citer l'hexadécényl succinate 18 OE (composé de formule I avec R=hexadécényl, E=(C₂H₄O)ₙ, n=18), l'hexadécényl succinate 45 OE (composé de formule I avec R=hexadécényl, E=(C₂H₄O)ₙ, n=45), le dihexadécényl succinate 18 OE (composé de formule Il avec R=R'=hexadécényl, E=(C₂H₄O)ₙ, n=18), le dihexadécényl succinate de glucose 10 OE (composé de formule Il avec R=R'=hexadécényl, E= glucose oxyéthyléné comportant 10 groupes oxyéthylénés), le dihexadécényl succinate de glucose 20 OE (composé de formule II avec R=R'=hexadécényl, E=glucose oxyéthyléné comportant 20 groupes oxyéthylénés), le dioctadécényl-succinate de méthyl glucose 20 OE (composé de formule Il avec R=R'=octadécényl, E= méthyl glucose oxyéthyléné comportant 20 groupes oxyéthylénés), et leurs mélanges.

Selon son caractère plus hydrophile ou plus lipophile, le tensioactif peut être introduit dans la phase aqueuse ou dans la phase huileuse de la nanoémulsion.

Selon l'invention, la quantité de lipide amphiphile va de 0,2 à 15 % en poids et de préférence de 1 à 8 % en poids par rapport au poids total de la nanoémulsion.

Le rapport en poids de la quantité de la phase huileuse sur la quantité de lipide amphiphile va de 1 à 10 et, de préférence, de 1,2 à 6. On entend ici par "quantité de phase huileuse" la quantité totale des constituants de cette phase sans inclure la quantité de lipide amphiphile.

Selon une forme particulière de réalisation de l'invention, la nanoémulsion de l'invention peut contenir en outre un ou plusieurs lipides amphiphiles ioniques additionnels différents de ceux décrits ci-dessus. Leur ajout, comme additif, a notamment pour objectif d'améliorer encore la stabilité de la dispersion.

Les lipides amphiphiles ioniques additionnels pouvant être utilisés dans les nanoémulsions de l'invention sont choisis de préférence dans le groupe formé par les lipides amphiphiles cationiques, amphotères et les lipides amphiphiles anioniques choisis parmi:
- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et di-sodiques comme le sel disodique de l'acide N-stéaroyl L-glutamique commercialisé sous la dénomination Acylglutamate HS21 par la société AJINOMOTO;
- les sels de sodium de l'acide phosphatidique;
- les phospholipides
- les dérivés alkylsulfoniques notamment de formule: 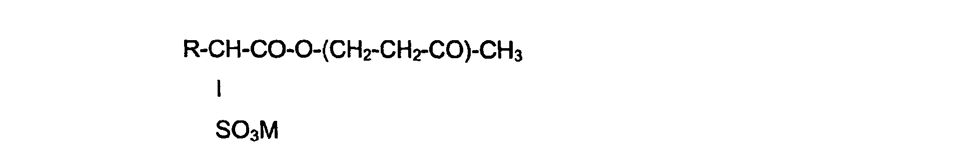
dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium.

Les lipides amphiphiles cationiques, utilisés dans les nanoémulsions de l'invention, sont choisis, de préférence, dans le groupe formé par les sels d'ammonium quaternaire, les amines grasses et leurs sels.

Les sels d'ammonium quaternaires sont par exemple :
- ceux qui présentent la formule générale (IV) suivante: 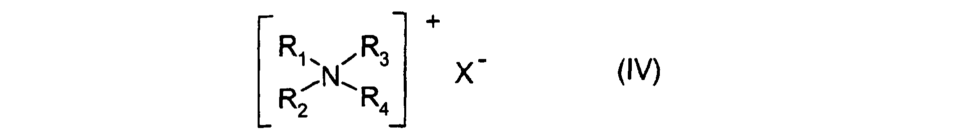
dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (V) suivante: 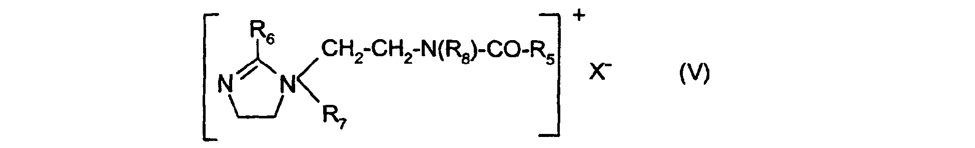
dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁₋C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
- les sels de diammonium quaternaire de formule (VI): 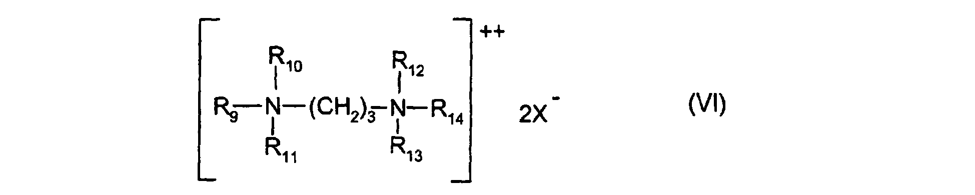
dans laquelle R₉ désigne un radical aliphatique comportant environ de 16 à 30 atomes de carbone, Rio, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester

Les sels d'ammonium quaternaire contenant au moins une fonction ester utilisables selon l'invention sont par exemple ceux de formule (VII) suivante: 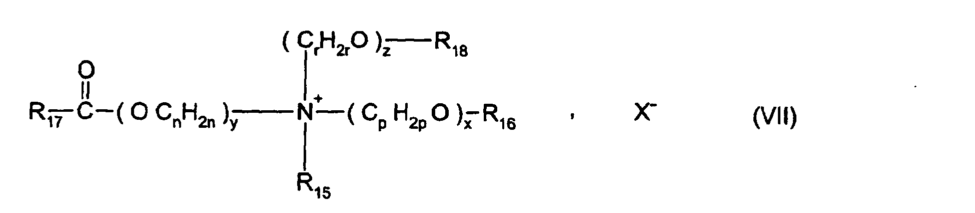 dans laquelle :
- R₁₅ est choisi parmi les radicaux alkyles en C₁-C₆ et les radicaux hydroxyalkyles ou dihydroxyalkyles en C₁-C₆;
- R₁₆ est choisi parmi:
   - le radical 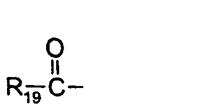
   - les radicaux R₂₀ hydrocarbonés en C₁-C₂₂ linéaires ou ramifiés, saturés ou insaturés,
   - l'atome d'hydrogène,
- R₁₈ est choisi parmi :
   - le radical 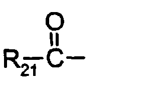
   - les radicaux R₂₂ hydrocarbonés en C₁-C₆ linéaires ou ramifiés, saturés ou insaturés,
- l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6;
- y est un entier valant de 1 à 10;
- x et z, identiques ou différents, sont des entiers valant de 0 à 10;
- X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement les sels d'ammonium de formule (VII) dans laquelle :
- R₁₅ désigne un radical méthyle ou éthyle,
- x et y sont égaux à 1;
- z est égal à 0 ou 1 ;
- n, p et r sont égaux à 2 ;
- R₁₆ est choisi parmi :
   - le radical 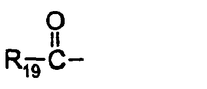
   - les radicaux méthyle, éthyle ou hydrocarbonés en C₁₄-C₂₂
   - l'atome d'hydrogène ;
- R₁₈ est choisi parmi:
   - le radical 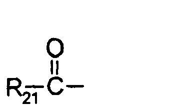
   - l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.
Avantageusement, les radicaux hydrocarbonés sont linéaires.

On peut citer par exemple les composés de formule (VII) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

La composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60% de méthylsulfate de diacyloxyéthyl hydroxyéthyl méthyl ammonium et 15 à 30% de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (IV) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK.

Selon l'invention, le chlorure de béhényltriméthylammonium est le sel d'ammonium quaternaire le plus particulièrement préféré.

Lorsque la nanoémulsion contient un ou plusieurs lipides amphiphiles additionnels cationiques ou anioniques, ils sont présents dans les nanoémulsions de l'invention, de préférence, dans des concentrations allant de 0,01 à 10% en poids par rapport au poids total de la nanoémulsion et plus particulièrement de 0,2 à 5% en poids.

Les huiles pouvant être utilisées dans les nanoémulsions de l'invention sont choisies préférentiellement dans le groupe formé par :
- les huiles animales ou végétales formées par des esters d'acides gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- les huiles de synthèse
- les huiles minérales telles que l'hexadécane et l'huile de paraffine ;
- les huiles halogénés, notamment des fluorocarbures tels que des fluoramines par exemple la perfluorotributylamine, des hydrocarbures fluorés, par exemple le perfluorodécahydronaphtalène, des fluoroesters et des fluoroethers;
- les esters d'acide minéral et d'un alcool ;
- les esters d'acides carboxyliques liquides;
- les silicones volatiles ou non volatiles.

Les huiles de synthèse sont notamment les polyoléfines en particulier les poly-α-oléfines et plus particulièrement:
- de type polybutène, hydrogéné ou non, et de préférence polyisobutène, hydrogéné ou non.

On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.

Les esters d'acides carboxyliques liquides peuvent être mono, di, tri ou tétracarboxyliques. Le nombre total de carbone des esters est généralement supérieur ou égal à 10 et de préférence inférieure à 100 et plus particulièrement inférieur à 80.

Les esters d'acides monocarboxyliques sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant généralement supérieur ou égal à 10.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

Parmi les esters cités ci-dessus, on préfère utiliser le palmitate d'éthyle, le palmitate d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Les nanoémulsions conformes à l'invention comportent une quantité d'huile allant de préférence, de 2 à 40% en poids par rapport au poids total de l'émulsion et plus particulièrement de 4 à 30% en poids et préférentiellement de 8 à 20% en poids.

Selon un mode de réalisation préféré en particulier pour les compositions capillaires, les compositions selon l'invention comprennent en outre au moins une silicone aminée.

Dans tout ce qui suit ou qui précède, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements amides, les radicaux acyloxy ou acyloxyalkyls, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon l'invention, on désigne par silicone aminée toute silicone comportant au moins une amine primaire, secondaire, tertiaire ou un groupement ammonium quaternaire. On peut ainsi citer:
(a) les polysiloxanes dénommés dans le dictionnaire CTFA "amodiméthicone" et répondant à la formule : 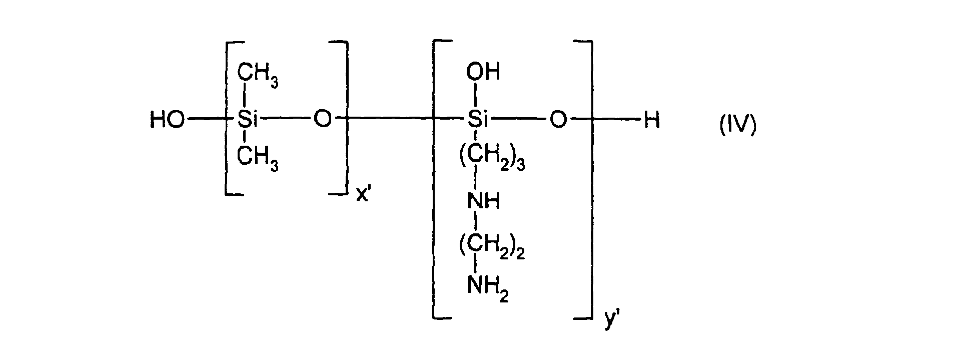 dans laquelle x' et y' sont des nombres entiers dépendant du poids moléculaire, généralement tels que ledit poids moléculaire moyen en poids est compris entre 5 000 et 500 000 environ ;
(b) les silicones aminées répondant à la formule:

   R'ₐG₃₋ₐ-Si(OSiG₂)ₙ-( OSiG_{b}R'_{2-b})ₘ-O-SiG₃₋ₐ-R'ₐ (V)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quaternisé choisi parmi les groupements :
      -NR"-CH₂-CH₂-N'(R")₂
      -N(R")₂
      -N^{⊕}(R")₃ A⁻
      -NH^{⊕}(R")₂ A⁻
      -NH₂^{⊕}ED(R") A⁻
      -N(R")-CH₂-CH₂-N® R" H₂ A⁻,
   dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.
   Un produit correspondant à cette définition est la silicone dénommée "triméthylsilylamodiméthicone", répondant à la formule: 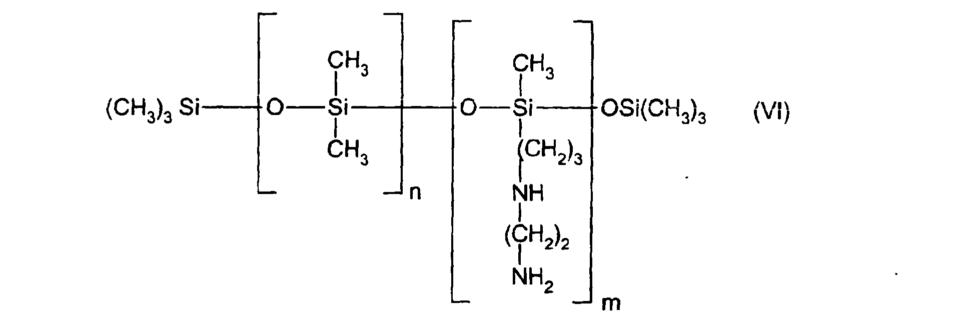 dans laquelle n et m ont les significations données ci-dessus (cf formule V).
   De tels polymères sont décrits par exemple dans la demande de brevet EP-A-95238.
(c) les silicones aminées répondant à la formule: 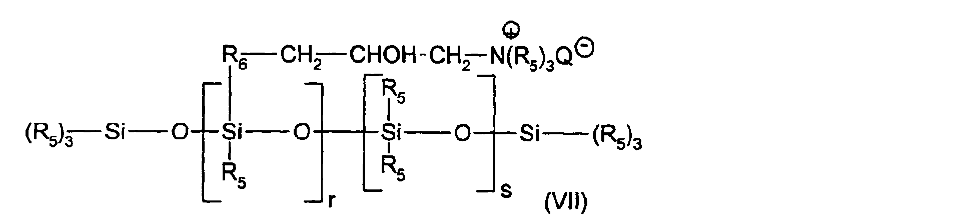 dans laquelle
   R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   Q est un anion tel qu'un ion halogénure, notamment chlorure ou un sel d'acide organique (acétate ...);
   r représente une valeur statistique moyenne de 2 à 20 et en particulier de 2 à 8;
   s représente une valeur statistique moyenne de 20 à 200 et en particulier de 20 à 50.
   De tels silicones aminées sont décrites plus particulièrement dans le brevet US 4 185 087.
   Une silicone entrant dans cette classe est la silicone commercialisée par la société Union Carbide sous la dénomination "Ucar Silicone ALE 56.
d) les silicones ammonium quaternaire de formule: 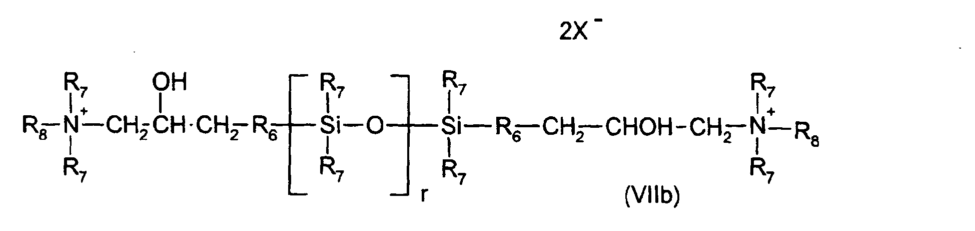 dans laquelle
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100;

Ces silicones sont par exemple décrites dans la demande EP-A-0530974.

Des silicones entrant dans cette classe sont les silicones commercialisées par la société GOLDSCHMIDT sous les dénominations ABIL QUAT 3270, ABIL QUAT 3272, ABIL QUAT 3474.

Selon l'invention, les silicones aminées peuvent se présenter sous formes d'huile, de solutions aqueuses, alcooliques ou hydroalcooliques, sous forme de dispersion ou d'émulsion.

Une forme de réalisation particulièrement intéressante est leur utilisation sous forme d'émulsions en particulier sous forme de microémulsions ou de nanoémulsions.

On peut utiliser par exemple le produit commercialisé sous la dénomination "Emulsion Cationique DC 929" par la Société Dow Corning qui comprend, outre l'amodiméthicone, un agent de surface cationique dérivés des acides gras du suif dénommé Tallowtrimonium(CTFA), en association avec un agent de surface non ionique connu sous la dénomination "Nonoxynol 10".

On peut également utiliser par exemple le produit commercialisé sous la dénomination "Emulsion Cationique DC 939" par la Société Dow Corning qui comprend, outre l'amodiméthicone, un agent de surface cationique le chlorure de triméthyl cétyl ammonium en association avec un agent de surface non ionique le tridéceth-12.

Un autre produit commercial utilisable selon l'invention est le produit commercialisé sous la dénomination "Dow Corning Q2 7224" par la Société Dow Corning comportant en association le triméthylsilylamodiméthicone de formule (IV), un agent de surface non ionique de formule: C₈H₁₇-C₆H₄-(OCH₂CH₂)ₙ-OH où n = 40 dénommé encore octoxynol-40,
un autre agent de surface non ionique de formule : C₁₂H₂₅-(OCH₂-CH₂)ₙ-OH où n = 6 encore dénommé isolaureth-6, et du glycol.

Avantageusement, la silicone aminée est présente à une concentration comprise entre 0,05 et 10% en poids par rapport au poids total de l'émulsion, de préférence entre 0,1 et 5 % en poids et plus particulièrement de 0,3 à 3% en poids.

Les émulsions conformes à la présente invention peuvent contenir des solvants notamment pour améliorer, si nécessaire, la transparence de la formulation.

Ces solvants sont choisis de préférence dans le groupe formé par :
- les alcools inférieurs en C₁-C₈ tels que l'éthanol;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.

Les solvants tels que ceux cités ci-dessus sont présents dans les émulsions de l'invention dans des concentrations allant, de préférence, de 0,01 à 30% en poids par rapport au poids total de l'émulsion.

En outre, l'utilisation des alcools tels que définis ci-dessus, à des concentrations supérieures ou égales à 5% en poids et de préférence supérieure à 15%, permet d'obtenir des émulsions sans conservateur.

Les émulsions de l'invention peuvent contenir des actifs hydrosolubles ou liposolubles, ayant une activité cosmétique ou dermopharmaceutique. Les actifs liposolubles sont dans les globules huileux de l'émulsion, tandis que les actifs hydrosolubles sont dans la phase aqueuse de l'émulsion. On peut citer, à titre d'exemples d'actif, les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines telles que le panthénol, le palmitate de vitamine A, la niacinamide, l'ergocalciférol, les anti oxydants, les huiles essentielles, les humectants, les filtres solaires siliconés ou non, des agents conservateurs, des séquestrants, des adoucissants, des colorants, des agents modificateurs de viscosité, des agents modificateurs de mousse, des stabilisateurs de mousse, des agents nacrants, des pigments, des agents hydratants, des agents antipelliculaires, des agents antiséborrhéiques, des protéines, des céramides, des pseudocéramides, des acides gras à chaînes linéaires ou ramifiées en C₁₆-C₄₀ tels que l'acide méthyl-18 eicosanoique, des plastifiants, des hydroxyacides, des électrolytes, des polymères en particulier cationiques et des parfums.

Les globules d'huile des émulsions de l'invention, ont de préférence une taille moyenne allant de 20 à 150 nm, plus préférentiellement de 30 à 100 nm et encore plus particulièrement de 40 à 80 nm.

Les nanoémulsions selon l'invention ont généralement un aspect transparent à bleuté. Leur transparence se mesure par un coefficient de transmittance à 600 nm allant de 10 à 90 % ou bien par une turbidité allant de 60 à 600 NTU et de préférence de 70 à 400 NTU, turbidité mesurée au turbidimètre portatif HACH - Modèle 2100 P.

Les nanoémulsions de l'invention peuvent être obtenues par un procédé, caractérisé par le fait qu'on mélange la phase aqueuse et la phase huileuse , sous agitation vive, à une température ambiante inférieure à 45°C puis qu'on effectue une homogénéisation haute pression à une pression supérieure à 5.10⁷ Pa et de préférence allant de 6.10⁷ à 18.10⁷ Pa. Un tel procédé permet de réaliser, à température ambiante, des nanoémulsions compatibles avec des composés actifs thermosensibles, et pouvant contenir des quantités importantes d'huiles et notamment des parfums qui renferment des corps gras, sans les dénaturer.

Un autre objet de l'invention consiste en une composition à usage topique telle qu'une composition cosmétique ou dermopharmaceutique, caractérisée par le fait qu'elle est constituée par une nanoémulsion telle que définie précédemment ou qu'elle comprend une telle nanoémulsion.

Les compositions conformes à l'invention peuvent être utilisées pour le lavage, le nettoyage et le démaquillage des matières kératiniques telles que les cheveux, la peau, les cils, les sourcils, les ongles, les muqueuses.

Les compositions de l'invention peuvent plus particulièrement se présenter sous forme de shampooings, d'après-shampooings à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage.

Les compositions peuvent être également des lotions de mise en plis, des lotions pour le brushing, des compositions de fixation (laques) et de coiffage telles que par exemple des gels ou des mousses. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

Les compositions conformes à l'invention peuvent être utilisées pour le soin et/ou le maquillage des matières kératiniques telles que les cheveux, la peau du corps et/ou du visage, les cils, les sourcils, les ongles, les muqueuses.

Un autre objet de l'invention est l'utilisation des émulsions telles que définies ci-dessus comme base de produits de soin et/ou de maquillage et/ou démaquillage pour la peau du corps et/ou du visage et/ou le cuir chevelu et/ou les cheveux et/ou les ongles et/ou les cils et/ou les sourcils et/ou les muqueuses (par exemple les lèvres), tels que des lotions, des sérums, des laits, des crèmes, des eaux de toilette.

Enfin, l'invention porte également sur un procédé non-thérapeutique de soin de la peau, des cheveux, des cils, des sourcils, des ongles, des muqueuses ou du cuir chevelu, caractérisé par le fait qu'on applique sur la peau, les cheveux, les cils, les sourcils, les ongles, les muqueuses ou le cuir chevelu une nanoémulsion telle que définie ci-dessus.

Les exemples qui suivent, permettront de mieux comprendre l'invention.

### EXEMPLES

Le mode opératoire suivant est mis en oeuvre :
- dans une première phase A, on homogénéise les lipides amphiphiles non ioniques et cationiques avec l'huile et les actifs et adjuvants lipophiles à une température d'environ 80°C; On laisse refroidir sous agitation raclante jusqu'à 50°C.
- on ajoute alors le parfum, le conservateur le cyclométhicone, on poursuit le refroidissement jusqu'à 30°C.
- dans une seconde phase B, on mélange 65% de l'eau et les actifs et adjuvants solubles ou dispersibles dans l'eau à une température de 20 à 30°C ;
- on a préparé une troisième phase C contenant 35% de l'eau et l'ester de PEG. On fait fondre à 80°C puis on refroidit à 60°C.
- puis, on mélange les phases A et B à l'aide d'un homogénéisateur à turbine puis on homogénéise à l'aide d'un homogénéisateur à haute pression du type Soavi-Niro à une pression de 1200 bars, en 4 passages en maintenant la température du produit en dessous d'environ 35°C.
   A température ambiante, on ajoute sous agitation la phase C.

### EXEMPLE 1 : Nanoémulsion

### Première phase A:

### Phase A:

- Isostéarate de PEG-400, vendu par la société UNICHEMA 2 g
- Chlorure de béhényltriméthylammonium à 80%MA (GENAMIN DDMP de GOLDSCHMIDT) 2 g (1,6 g MA)
- Huile d'avocat 5,25 g
- Huile de jojoba 5,25 g
- Parfum qs
- Ethanol (95-96°) 5g
- Conservateur qs
- Cyclopentadiméthylsiloxane (DC245 de DOW CORNING) 3,5 g

### Phase B:

- Microémulsion de triméthylsilylamodiméthicone à 20%MA commercialisée sous la dénomination SME 253 par la société GENERAL ELECTRIC 6 g (1,2 g MA)
- Propylèneglycol 5 g
- Monolaurate de sorbitane oxyéthyléné à 20 moles d'oxyde d'éthylène (TWEEN 20 de ICI) 0,5 g
- Eau déminéralisée 42 g
- Glycérine 5 g

### Phase C:

- Alkyl(C₈₋₃₀)hydroxyéthyl-cellulose quatemisée, (Quatrisoft LM 200 de Amerchol) 1 g
- Eau 22 g

On obtient une émulsion dont la taille des globules d'huile est d'environ 63 nm. Cette composition est stable au stockage 2 mois à température ambiante et à 45°C.

La composition présente une turbidité de 375 NTU et une viscosité de 650 mPa.s (cP).

La viscosité est mesurée avec un rhéomètre de type Rhéomat 108 avec un gradient de vitesse de 200s⁻¹ à 25°C (mobile 3).

Les cheveux traités avec cette composition sont faciles à démêler, doux et brillants.

Si l'on remplace le Quatrisoft LM 200 par la même quantité de Carbopol Ultrez, on obtient une composition qui n'est pas épaissie, qui n'est pas transparente (turbidité > 1000 NTU) et qui n'est pas stable au stockage.

## Revendications

1. Nanoémulsion huile-dans-eau comportant une phase huileuse dispersée dans une phase aqueuse dont les globules d'huile ont une taille moyenne en nombre inférieure à 150 nm, **caractérisée par le fait qu'**elles comprennent au moins une huile, au moins un lipide amphiphile et au moins un polymère cationique, comportant au moins une séquence hydrophobe et au moins une séquence hydrophile et que le rapport pondéral de la quantité d'huile sur la quantité de lipide amphiphile est compris entre 1 et 10.

2. Nanoémulsion selon la revendication 1, **caractérisée en ce que** le rapport pondéral de la quantité d'huile sur la quantité de lipide amphiphile va de 1,2 à 6.

3. Nanoémulsion selon l'une quelconque des revendications précédentes, **caractérisée en ce que** les globules d'huile ont une taille moyenne allant de 30 à 100 nm.

4. Nanoémulsion selon l'une quelconque des revendications précédentes, **caractérisée en ce que** ledit polymère cationique est hydrosoluble ou hydrodispersible.

5. Nanoémulsion selon l'une quelconque des revendications précédentes, **caractérisée en ce que** ledit polymère cationique comporte au moins deux séquences hydrophobes.

6. Nanoémulsion selon l'une quelconque des revendications précédentes, **caractérisée en ce que** la ou les séquences hydrophobes sont des chaînes grasses ayant de 6 à 30 atomes de carbone, des groupements divalents aliphatiques des groupements divalents cycloaliphatiques, ou des groupements divalents aromatiques.

7. Nanoémulsion selon l'une quelconque des revendications précédentes, **caractérisée en ce que** la ou les séquences hydrophobes sont des chaînes hydrocarbonées telles que alkyle, arylalkyle, alkylaryle ou alcényle.

8. Nanoémulsion selon l'une quelconque des revendications précédentes, **caractérisée en ce que** la ou les séquences hydrophiles sont choisis parmi un polyoxyde d'éthylène, un polysaccharide, un polyamide notamment polyacrylamide, un polyester et leurs mélanges.

9. Nanoémulsion selon l'une quelconque des revendications précédentes, **caractérisée en ce que** la ou les liaisons entre séquence hydrophobe et hydrophile sont choisies parmi les liaisons de type ester, éther, urée, amide ou uréthane et leurs mélanges.

10. Nanoémulsion selon l'une quelconque des revendications précédentes, **caractérisée en ce que** le rapport (en poids) de la (ou les) séquence(s) hydrophile(s) sur la (ou les) séquence(s) hydrophobe(s) du polymère est compris entre 10/1 et 1000/1.

11. Nanoémulsion selon l'une quelconque des revendications précédentes, **caractérisée par le fait que** ledit polymère cationique est choisi parmi les dérivés de cellulose quaternisée et les polyacrylates à groupements latéraux aminés.

12. Nanoémulsion selon l'une quelconque des revendications précédentes, **caractérisée en ce que** la quantité de polymères cationiques comportant au moins une séquence hydrophobe et au moins une séquence hydrophile est comprise entre 0,1 et 20% et de préférence entre 0,5 et 10% en poids par rapport au poids total de la composition.

13. Nanoémulsion selon l'une quelconque des revendications précédentes, **caractérisée en ce qu'**elle comprend au moins un lipide amphiphile non ionique et/ou au moins un lipide amphiphile anionique.

14. Nanoémulsion selon la revendication précédente, **caractérisée en ce que** les lipides amphiphiles non-ioniques de l'invention sont choisis parmi:
1/- les tensioactifs siliconés,
2/- les lipides amphiphiles fluides à température inférieure ou égale à 45°C choisis parmi les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée,
3/- les esters mixtes d'acide gras ou d'alcool gras, d'acide carboxylique et de glycérol,
4/- les esters d'acide gras et de sucre et les éthers d'alcool gras et de sucre,
5/- les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés,
6/- les copolymères blocs d'oxyde d'éthylène (A) et d'oxyde de propylène (B).

15. Nanoémulsion selon l'une quelconque des revendications 13 ou 14, **caractérisée en ce que** les lipides amphiphiles non ioniques sont choisis parmi :
- l'isostéarate de polyéthylèneglycol (8 moles d'oxyde d'éthylène),
- l'isostéarate de diglycéryle,
- le monolaurate et le monostéarate de polyglycérol comportant 10 unités de glycérol ,
- l'oléate de sorbitane,
- l'isostéarate de sorbitane.

16. Nanoémulsion selon l'une quelconque des revendications 13 à 15, **caractérisée en ce que** les lipides amphiphiles anioniques sont choisis parmi:
- les citrates d'alkyléther,
- les alkényl succinates alkoxylés,
- les alkényl succinates de glucose alkoxylés,
- les alkényl succinates de méthylglucose alkoxylés.

17. Nanoémulsion selon l'une quelconque des revendications précédentes, **caractérisée en ce que** la quantité de lipide amphiphile va de 0,2 à 15 % en poids et de préférence de 1 à 8 % en poids par rapport au poids total de la nanoémulsion.

18. Nanoémulsion selon l'une quelconque des revendications précédentes, **caractérisée en ce qu'**elle comprend en outre au moins un lipide amphiphile ionique additionnel choisis dans le groupe formé par les lipides amphiphiles cationiques et les lipides amphiphiles anioniques choisis parmi :
- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et disodiques comme le sel disodique de l'acide N-stéaroyl L-glutamique;
- les sels de sodium de l'acide phosphatidique;
- les phospholipides
- les dérivés alkylsulfoniques notamment de formule: 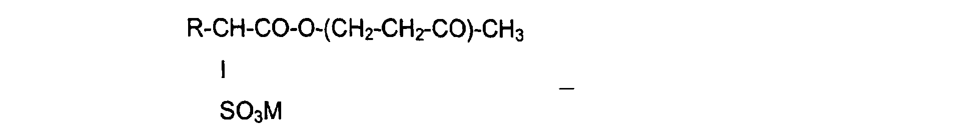
dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium.

19. Nanoémulsion selon la revendication précédente, **caractérisée en ce que** ledit lipide amphiphile additionnel cationique est choisi parmi:
A) les sels d'ammonium quaternaires de la formule générale (IV) suivante: 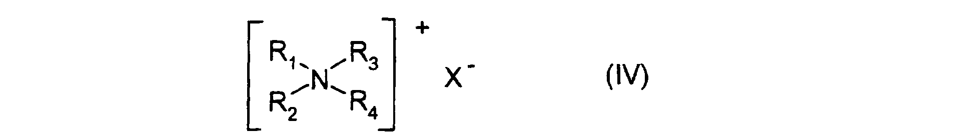 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
B) - les sels d'ammonium quaternaire de l'imidazolinium, comme par exemple celui de formule (V) suivante: 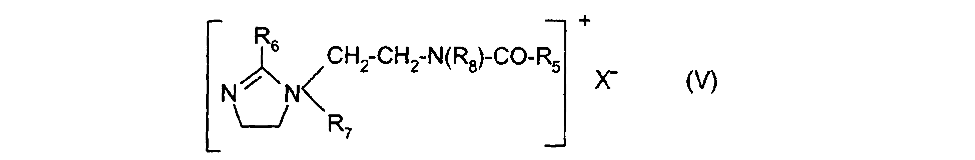 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁₋C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates.
C) - les sels de diammonium quaternaire de formule (VI): 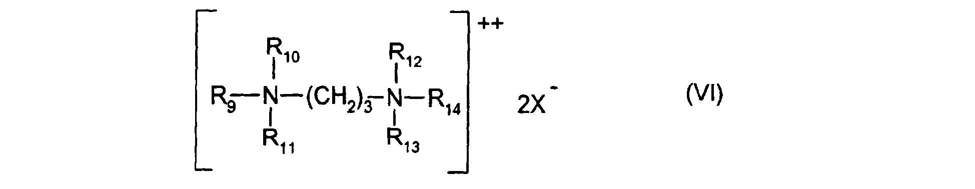 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (VII) suivante: 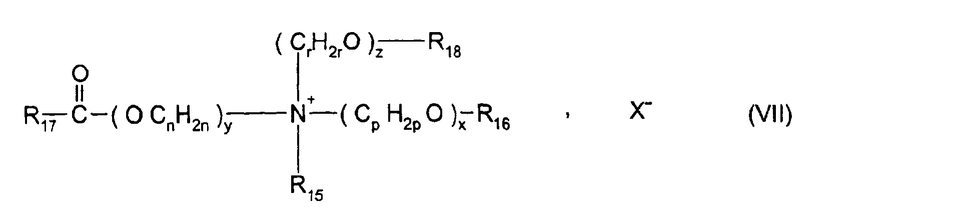
dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
- le radical 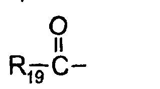
- les radicaux R₂₀ hydrocarbonés en C₁-C₂₂ linéaires ou ramifiés, saturés ou insaturés,
- l'atome d'hydrogène,
- R₁₈ est choisi parmi:
- le radical 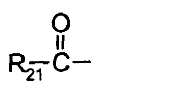
- les radicaux R₂₂ hydrocarbonés en C₁-C₆ linéaires ou ramifiés, saturés ou insaturés,
- l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6;
- y est un entier valant de 1 à 10;
- x et z, identiques ou différents, sont des entiers valant de 0 à 10;
- X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.

20. Nanoémulsion selon la revendication précédente, **caractérisée par le fait que** les dits tensioactifs cationiques de formule (IV) sont choisis parmi les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, le chlorure de stéaramidopropyldiméthyl (myristyl acétate) ammonium.

21. Nanoémulsion selon l'une quelconque des revendications 19 ou 20, **caractérisée par le fait que** ledit tensioactif cationique est choisi parmi les sels de béhényl triméthyl ammonium, les sels de stéaramidopropyl diméthyl (myristylacétate) ammonium.

22. Nanoémulsion selon l'une quelconque des revendications 18 à 21, **caractérisée en ce que** ledit lipide amphiphile additionnel cationique ou anionique est présent dans les nanoémulsions dans des concentrations allant de 0,01 à 10% en poids par rapport au poids total de la nanoémulsion et plus particulièrement de 0,2 à 5% en poids.

23. Nanoémulsion selon l'une quelconque des revendications précédentes, **caractérisée en ce que** l'huile est choisie parmi les huiles végétales, les huiles animales, les huiles de synthèse, les huiles minérales, les huiles halogénées, les esters d'acide minéral et d'un alcool, les esters d'acides carboxyliques liquides et les silicones.

24. Nanoémulsion selon l'une quelconque des revendications 1 à 23, **caractérisée en ce que** la quantité d'huile va de 2 à 40 % en poids et de préférence de 4 à 30 % en poids par rapport au poids total de la nanoémulsion.

25. Nanoémulsion selon l'une quelconque des revendications 1 à 24, **caractérisée par le fait qu'**elle comprend un actif cosmétique ou dermopharmaceutique, hydrosoluble ou liposoluble.

26. Nanoémulsion selon l'une quelconque des revendications 1 à 25, **caractérisée en ce qu'**elle a une turbidité allant de 60 à 600 NTU.

27. Composition cosmétique à usage topique, **caractérisée par le fait qu'**elle est constituée d'une nanoémulsion ou comprend une nanoémulsion selon l'une quelconque des revendications 1 à 26.

28. Utilisation d'une nanoémulsion telle que définie selon l'une quelconque des revendications 1 à 26 comme ou dans des produits de soin et/ou de lavage et/ou de maquillage et/ou démaquillage de la peau du corps et/ou du visage et/ou des muqueuses et/ou du cuir chevelu et/ou des cheveux et/ou des ongles, et/ou des cils et/ou des sourcils.

29. Procédé de traitement non-thérapeutique de la peau, des cheveux, des muqueuses, des ongles, des cils, des sourcils et/ou du cuir chevelu, **caractérisé par le fait qu'**on applique sur la peau, les cheveux, les muqueuses, les ongles, les cils, les sourcils ou le cuir chevelu une nanoémulsion selon l'une quelconque des revendications 1 à 26 ou une composition selon la revendication 27.

30. Procédé pour épaissir les nanoémulsions huile-dans-eau ayant des globules d'huile dont la taille moyenne en nombre est inférieure à 150 nm, dans lequel on ajoute à ladite composition un polymère cationique qui comporte au moins une séquence hydrophobe et au moins une séquence hydrophile.

## Claims

1. Oil-in-water nanoemulsion comprising an oily phase dispersed in an aqueous phase, the oil globules of which have a number-average size of less than 150 nm, **characterized in that** they comprise at least one oil, at least one amphiphilic lipid and at least one cationic polymer comprising at least one hydrophobic block and at least one hydrophilic block and **in that** the weight ratio of the amount of oil to the amount of amphiphilic lipid is between 1 and 10.

2. Nanoemulsion according to Claim 1, **characterized in that** the weight ratio between the amount of oil and the amount of amphiphilic lipid ranges from 1.2 to 6.

3. Nanoemulsion according to either of the preceding claims, **characterized in that** the oil globules have an average size ranging from 30 to 100 nm.

4. Nanoemulsion according to any one of the preceding claims, **characterized in that** the said cationic polymer is water-soluble or water-dispersible.

5. Nanoemulsion according to any one of the preceding claims, **characterized in that** the said cationic polymer comprises at least two hydrophobic blocks.

6. Nanoemulsion according to any one of the preceding claims, **characterized in that** the hydrophobic block(s) is (are) fatty chains containing from 6 to 30 carbon atoms, divalent aliphatic groups, divalent cycloaliphatic groups or divalent aromatic groups.

7. Nanoemulsion according to any one of the preceding claims, **characterized in that** the hydrophobic block(s) is (are) hydrocarbon-based chains such as alkyl, arylalkyl, alkylaryl or alkenyl.

8. Nanoemulsion according to any one of the preceding claims, **characterized in that** the hydrophilic block(s) is (are) chosen from a polyethylene oxide, a polysaccharide, a polyamide, in particular polyacrylamide, and a polyester, and mixtures thereof.

9. Nanoemulsion according to any one of the preceding claims, **characterized in that** the bond(s) between a hydrophobic and a hydrophilic block is (are) chosen from bonds of ester, ether, urea, amide and urethane type, and mixtures thereof.

10. Nanoemulsion according to any one of the preceding claims, **characterized in that** the ratio (by weight) of the hydrophilic block(s) to the hydrophobic block(s) of the polymer is between 10/1 and 1000/1.

11. Nanoemulsion according to any one of the preceding claims, **characterized in that** the said cationic polymer is chosen from quaternized cellulose derivatives and polyacrylates containing amine side groups.

12. Nanoemulsion according to any one of the preceding claims, **characterized in that** the amount of cationic polymers comprising at least one hydrophobic block and at least one hydrophilic block is between 0.1% and 20% and preferably between 0.5% and 10% by weight relative to the total weight of the composition.

13. Nanoemulsion according to any one of the preceding claims, **characterized in that** it comprises at least one nonionic amphiphilic lipid and/or at least one anionic amphiphilic lipid.

14. Nanoemulsion according to the preceding claim, **characterized in that** the nonionic amphiphilic lipids of the invention are chosen from:
1/- silicone surfactants,
2/- amphiphilic lipids that are fluid at a temperature of less than or equal to 45°C, chosen from the esters of at least one polyol chosen from the group formed by polyethylene glycol comprising from 1 to 60 ethylene oxide units, sorbitan, glycerol comprising from 2 to 30 ethylene oxide units, polyglycerols comprising from 2 to 15 glycerol units, and of at least one fatty acid comprising at least one saturated or unsaturated, linear or branched C₈-C₂₂ alkyl chain,
3/- mixed esters of fatty acid or of fatty alcohol, of carboxylic acid and of glycerol,
4/- fatty acid esters of sugars and fatty alcohol ethers of sugars,
5/- surfactants that are solid at a temperature of less than or equal to 45°C, chosen from fatty esters of glycerol, fatty esters of sorbitan and oxyethylenated fatty esters of sorbitan, ethoxylated fatty ethers and ethoxylated fatty esters,
6/- block copolymers of ethylene oxide (A) and of propylene oxide (B).

15. Nanoemulsion according to either of Claims 13 and 14, **characterized in that** the nonionic amphiphilic lipids are chosen from:
- polyethylene glycol isostearate (8 mol of ethylene oxide),
- diglyceryl isostearate,
- polyglyceryl monolaurate and monostearate comprising 10 glycerol units,
- sorbitan oleate,
- sorbitan isostearate.

16. Nanoemulsion according to any one of Claims 13 to 15, **characterized in that** the anionic amphiphilic lipids are chosen from:
- alkyl ether citrates,
- alkoxylated alkenyl succinates,
- alkoxylated glucose alkenyl succinates,
- alkoxylated methylglucose alkenyl succinates.

17. Nanoemulsion according to any one of the preceding claims, **characterized in that** the amount of amphiphilic lipid ranges from 0.2% to 15% by weight and preferably from 1% to 8% by weight relative to the total weight of the nanoemulsion.

18. Nanoemulsion according to any one of the preceding claims, **characterized in that** it also comprises at least one additional ionic amphiphilic lipid chosen the group formed by cationic amphiphilic lipids and anionic amphiphilic lipids chosen from:
- alkaline salts of dicetyl phosphate and of dimyristyl phosphate;
- alkaline salts of cholesteryl sulphate;
- alkaline salts of cholesteryl phosphate;
- lipoamino acids and salts thereof, such as monosodium and disodium acylglutamates, for instance the disodium salt of N-stearoyl-L-glutamic acid;
- sodium salts of phosphatidic acid;
- phospholipids
- alkylsulphonic derivatives in particular of formula: 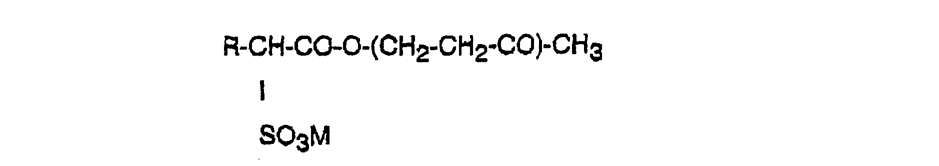
in which R represents C₁₆-C₂₂ alkyl radicals, in particular the radicals C₁₆H₃₃ and C₁₈H₃₇, taken as a mixture or separately, and M is an alkali metal or alkaline-earth metal such as sodium.

19. Nanoemulsion according to the preceding claim, **characterized in that** the said additional cationic amphiphilic lipid is chosen from:
A) the quaternary ammonium salts of the general formula (IV) below: 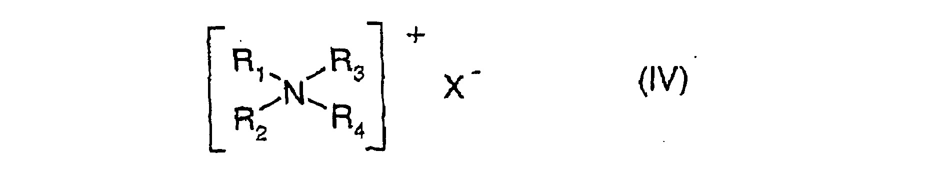 in which the radicals R₁ to R₄, which may be identical or different, represent a linear or branched aliphatic radical containing from 1 to 30 carbon atoms, or an aromatic radical such as aryl or alkylaryl. The aliphatic radicals can comprise hetero atoms such as, in particular, oxygen, nitrogen, sulphur and halogens. The aliphatic radicals are chosen, for example, from alkyl, alkoxy, polyoxy(C₂-C₆)alkylene, alkylamide, (C₁₂-C₂₂) alkylamido (C₂-C₆) alkyl, (C₁₂-C₂₂) alkylacetate and hydroxyalkyl radicals, containing from about 1 to 30 carbon atoms; X is an anion chosen from the group of halides, phosphates, acetates, lactates, (C₂-C₆)alkyl sulphates and alkyl- or akylarylsulphonates,
B) - the quaternary ammonium salts of imidazolinium, such as, for example, that of formula (V) below: 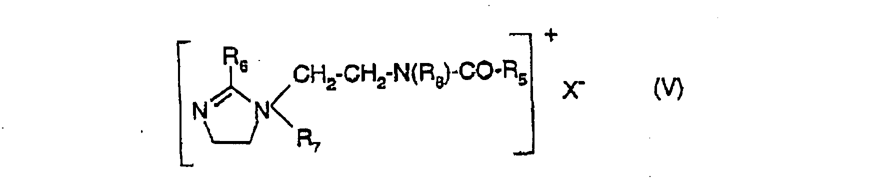 in which R₅ represents an alkenyl or alkyl radical containing from 8 to 30 carbon atoms, for example fatty acid derivatives of tallow, R₆ represents a hydrogen atom, a C₁-C₄ alkyl radical or an alkenyl or alkyl radical containing from 8 to 30 carbon atoms, R₇ represents a C₁-C₄ alkyl radical, R₈ represents a hydrogen atom or a C₁-C₄ alkyl radical, and X is an anion chosen from the group of halides, phosphates, acetates, lactates, alkyl sulphates, alkyl- or alkylarylsulphonates.
C) - the diquaternary ammonium salts of formula (VI) : 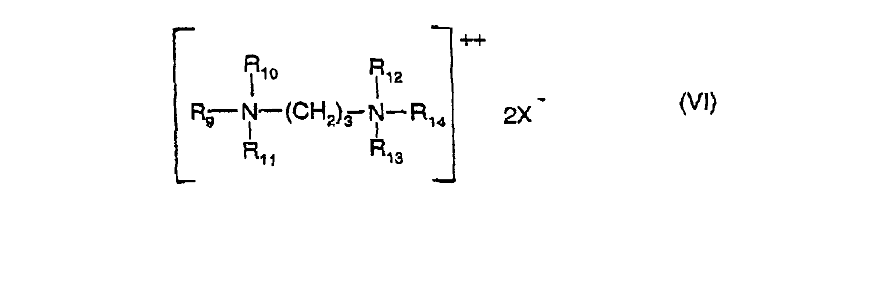 in which R₉ denotes an aliphatic radical containing from about 16 to 30 carbon atoms, R₁₀, R₁₁, R₁₂, R₁₃ and R₁₄, which may be identical or different, are chosen from hydrogen and an alkyl radical containing from 1 to 4 carbon atoms, and X is an anion chosen from the group of halides, acetates, phosphates, nitrates and methyl sulphates, Such diquaternary ammonium salts in particular comprise propanetallowdiammmonium dichloride.
D) - quaternary ammonium salts containing at least one ester function of formula (VII) below: 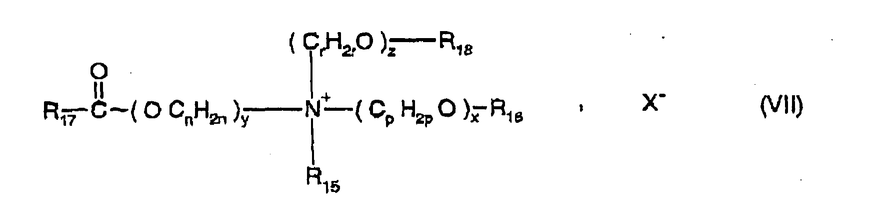
in which:
- R₁₅ is chosen from C₁-C₆ alkyl radicals and C₁-C₆ hydroxyalkyl or dihydroxyalkyl radicals;
- R₁₆ is chosen from:
- a radical 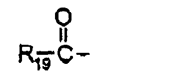
- linear or branched, saturated or unsaturated C₁-C₂₂ hydrocarbon-based radicals R₂₀,
- a hydrogen atom,
- R₁₈ is chosen from:
- a radical 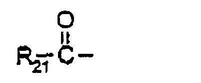
- linear or branched, saturated or unsaturated C₁-C₆ hydrocarbon-based radicals R₂₂,
- a hydrogen atom,
- R₁₇, R₁₉ and R₂₁, which may be identical or different, are chosen from linear or branched, saturated or unsaturated C₇-C₂₁ hydrocarbon-based radicals;
- n, p and r, which may be identical or different, are integers ranging from 2 to 6;
- y is an integer ranging from 1 to 10;
- x and z, which may be identical or different, are integers ranging from 0 to 10;
- X⁻ is a simple or complex, organic or inorganic anion;
with the proviso that the sum x + y + z is from 1 to 15, that when x is 0, then R₁₆ denotes R₂₀ and that when z is 0, then R₁₈ denotes R₂₂.

20. Nanoemulsion according to the preceding claim, **characterized in that** the said cationic surfactants of formula (IV) are chosen from tetraalkylammonium chlorides such as, for example, dialkyldimethylammonium or alkyltrimethylammonium chlorides, in which the alkyl radical contains from about 12 to 22 carbon atoms, in particular behenyltrimethylammonium chloride, distearyldimethylammonium chloride, cetyltrimethylammonium chloride, benzyldimethylstearylammonium chloride or stearamidopropyldimethyl(myristyl acetate)ammonium chloride.

21. Nanoemulsion according to either of Claims 19 and 20, **characterized in that** the said cationic surfactant is chosen from behenyltrimethylammonium salts and stearamidopropyldimethyl(myristyl acetate)ammonium salts.

22. Nanoemulsion according to any one of Claims 18 to 21, **characterized in that** said additional cationic or anionic amphiphilic lipid is present in the nanoemulsions in concentrations ranging from 0.01% to 10% by weight relative to the total weight of the nanoemulsion and more particularly from 0.2% to 5% by weight.

23. Nanoemulsion according to any one of the preceding claims, **characterized in that** the oil is chosen from plant oils, animal oils, synthetic oils, mineral oils, halogenated oils, esters of a mineral acid and of an alcohol, liquid carboxylic acid esters and silicones.

24. Nanoemulsion according to any one of Claims 1 to 23, **characterized in that** the amount of oil ranges from 2% to 40% by weight and preferably from 4% to 30% by weight relative to the total weight of the nanoemulsion.

25. Nanoemulsion according to any one of Claims 1 to 24, **characterized in that** it comprises a water-soluble or liposoluble cosmetic or dermo-pharmaceutical active agent.

26. Nanoemulsion according to any one of Claims 1 to 25, **characterized in that** it has a turbidity ranging from 60 NTU to 600 NTU.

27. Cosmetic composition for topical use, **characterized in that** it consists of a nanoemulsion or comprises a nanoemulsion according to any one of Claims 1 to 26.

28. Use of a nanoemulsion as defined according to any one of Claims 1 to 26, as or in care products and/or washing products and/or make-up products and/or make-up-removing products for body and/or facial skin and/or mucous membranes and/or the scalp and/or the hair and/or the nails and/or the eyelashes and/or the eyebrows.

29. Non-therapeutic process for treating the skin, the hair, mucous membranes, the nails, the eyelashes, the eyebrows and/or the scalp, **characterized in that** a nanoemulsion according to any one of Claims 1 to 26 or a composition according to Claim 27 is applied to the skin, the hair, mucous membranes, the nails, the eyelashes, the eyebrows or the scalp.

30. Process for thickening oil-in-water nanoemulsions containing oil globules whose number-average size is less than 150 nm, in which a cationic polymer which comprises at least one hydrophobic block and at least one hydrophilic block is added to said composition.

## Patentansprüche

1. 01-in-Wasser-Nanoemulsion, die eine in einer wässrigen Phase dispergierte Ölphase enthält, deren Öltröpfchen eine zahlenmittlere Größe unter 150 nm aufweisen, **dadurch gekennzeichnet, dass** sie mindestens ein Öl, mindestens ein amphiphiles Lipid und mindestens ein kationisches Polymer enthält, das mindestens eine hydrophobe Sequenz und mindestens eine hydrophile Sequenz aufweist, und **dadurch**, dass das Gewichtsverhältnis der Ölmenge und der Menge des amphiphilen Lipids im Bereich von 1 bis 10 liegt.

2. Nanoemulsion nach Anspruch 1, **dadurch gekennzeichnet, dass** das Gewichtsverhältnis der Ölmenge und der Menge des amphiphilen Lipids im Bereich von 1,2 bis 6 liegt.

3. Nanoemulsion nach einem der vorhergehenden Ansprüche, **dadurch gekennzeichnet, dass** die Öltröpfchen eine mittlere Größe von 30 bis 100 nm aufweisen.

4. Nanoemulsion nach einem der vorhergehenden Ansprüche, **dadurch gekennzeichnet, dass** das kationische Polymer in Wasser löslich oder in Wasser dispergierbar ist.

5. Nanoemulsion nach einem der vorhergehenden Ansprüche, **dadurch gekennzeichnet, dass** das kationische Polymer mindestens zwei hydrophobe Sequenzen besitzt.

6. Nanoemulsion nach einem der vorhergehenden Ansprüche, **dadurch gekennzeichnet, dass** die hydrophobe(n) Sequenz(en) Fettketten mit 6 bis 30 Kohlenstoffatomen, zweiwertige aliphatische Gruppen, zweiwertige cycloaliphatische Gruppen oder zweiwertige aromatische Gruppen sind.

7. Nanoemulsion nach einem der vorhergehenden Ansprüche, **dadurch gekennzeichnet, dass** die hydrophobe(n) Sequenz(en) Kohlenwasserstoffketten wie Alkyl, Arylalkyl, Alkylaryl oder Alkenyl sind.

8. Nanoemulsion nach einem der vorhergehenden Ansprüche, **dadurch gekennzeichnet, dass** die hydrophile(n) Sequenz(en) unter Polyethylenoxiden, Polysacchariden, Polyamiden und insbesondere Polyacrylamid, Polyestern und deren Gemischen ausgewählt sind.

9. Nanoemulsion nach einem der vorhergehenden Ansprüche, **dadurch gekennzeichnet, dass** die Bindung(en) zwischen der hydrophoben und der hydrophilen Sequenz unter den Bindungen vom Typ Ester, Ether, Harnstoff, Amid oder Urethan und deren Gemischen ausgewählt sind.

10. Nanoemulsion nach einem der vorhergehenden Ansprüche, **dadurch gekennzeichnet, dass** das (auf das Gewicht bezogene) Verhältnis der hydrophilen Sequenz(en) und der hydrophoben Sequenz(en) des Polymers im Bereich von 10/1 bis 1 000/1 liegt.

11. Nanoemulsion nach einem der vorhergehenden Ansprüche, **dadurch gekennzeichnet, dass** das kationische Polymer unter den Derivaten von quaternisierter Cellulose und Polyacrylaten mit aminierten Seitengruppen ausgewählt ist.

12. Nanoemulsion nach einem der vorhergehenden Ansprüche, **dadurch gekennzeichnet, dass** der Mengenanteil der kationischen Polymere, die mindestens eine hydrophobe Sequenz und mindestens eine hydrophile Sequenz besitzen, im Bereich von 0,1 bis 20 % und vorzugsweise 0,5 bis 10 Gew.-%, bezogen auf das Gesamtgewicht der Zusammensetzung, liegt.

13. Nanoemulsion nach einem der vorhergehenden Ansprüche, **dadurch gekennzeichnet, dass** sie mindestens ein nichtionisches amphiphiles Lipid und/oder mindestens ein anionisches amphiphiles Lipid enthält.

14. Nanoemulsion nach dem vorhergehenden Anspruch, **dadurch gekennzeichnet, dass** die erfindungsgemäßen nichtionischen amphiphilen Lipide ausgewählt sind unter:
1/- siliconierten grenzflächenaktiven Stoffen,
2/- bei einer Temperatur von 45 °C oder darunter fluiden amphiphilen Lipiden, die unter den Estern mindestens eines Polyols, das unter Polyethylenglykol mit 1 bis 60 Ethylenoxideinheiten, Sorbitan, Glycerin mit 2 bis 30 Ethylenoxideinheiten und Polyglycerinen mit 2 bis 15 Glycerineinheiten ausgewählt ist, und mindestens einer Fettsäure, die mindestens eine gesättigte oder ungesättigte, geradkettige oder verzweigte C₈₋₂₂-Alkylkette enthält, ausgewählt sind,
3/- gemischten Estern einer Fettsäure oder eines Fettalkohols, einer Carbonsäure und von Glycerin,
4/- Estern einer Fettsäure und eines Zuckers und Ethern eines Fettalkohols und eines Zuckers,
5/- bei einer Temperatur von 45 °C oder darunter festen grenzflächenaktiven Stoffen, die unter den Glycerinfettsäureestern, Sorbitanfettsäureestern und ethoxylierten Sorbitanfettsäureestern, ethoxylierten Fettethern und ethoxylierten Fettestern ausgewählt sind, und
6/- Blockcopolymeren von Ethylenoxid (A) und Propylenoxid (B).

15. Nanoemulsion nach einem der Ansprüche 13 oder 14, **dadurch gekennzeichnet, dass** die nichtionischen amphiphilen Lipide ausgewählt sind unter:
- Polyethylengtykolisostearat (8 mol Ethylenoxid),
- Diglycerylisostearat,
- Monolaurat und Monostearat von Polyglycerin mit 10 Glycerineinheiten,
- Sorbitanoleat und
- Sorbitanisostearat.

16. Nanoemulsion nach einem der Ansprüche 13 bis 15, **dadurch gekennzeichnet, dass** die anionischen amphiphilen Lipide ausgewählt sind unter:
- Alkylethereitraten,
- alkoxylierten Alkenylsuccinaten,
- alkoxylierten Glucosealkenylsuccinaten und
- alkoxylierten Methylglucosealkenylsuccinaten.

17. Nanoemulsion nach einem der vorhergehenden Ansprüche, **dadurch gekennzeichnet, dass** der Mengenanteil des amphiphilen Lipids im Bereich von 0,2 bis 15 Gew.-% und vorzugsweise 1 bis 8 Gew.-%, bezogen auf das Gesamtgewicht der Nanoemulsion, liegt.

18. Nanoemulsion nach einem der vorhergehenden Ansprüche, **dadurch gekennzeichnet, dass** sie ferner mindestens ein zusätzliches ionisches amphiphiles Lipid enthält, das unter den kationischen amphiphilen Lipiden und den anionischen amphiphilen Lipiden ausgewählt ist, welche ausgewählt sind unter:
- Alkalisalzen von Dicetyl- und Dimyristylphosphat;
- Alkalisalzen von Cholesterinsulfat;
- Alkalisalzen von Cholesterinphosphat;
- Lipoaminosäuren und deren Salzen, wie Mono- und Dinatriumacylglutamaten, wie dem Dinatriumsalz von N-Stearoyl-L-glutaminsäure;
- Natriumsalzen von Phosphatidsäure;
- Phospholipiden, und
- Alkylsulfonsäurederivaten insbesondere der folgenden Formel: 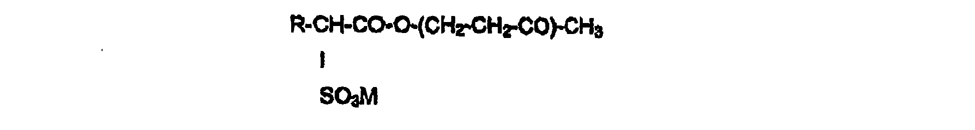 worin R C₁₆₋₂₂-Alkyl und insbesondere im Gemisch oder einzeln die Gruppen C₁₆H₃₃ und C₁₈H₃₇ bedeutet und M ein Alkalimetall oder Erdalkalimetall wie Natrium ist.

19. Nanoemulsion nach dem vorhergehenden Anspruch, **dadurch gekennzeichnet, dass** das zusätzliche kationische amphiphile Lipid ausgewählt ist unter:
A) quartären Ammoniumsalzen der folgenden allgemeinen Formel (IV): 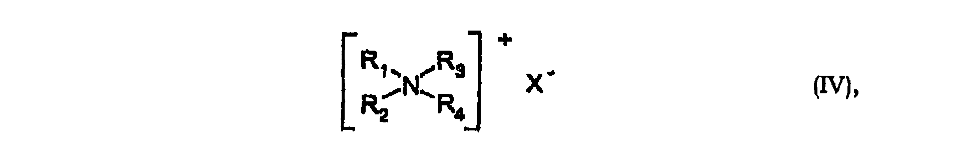 worin die Gruppen R₁ bis R₄, die gleich oder verschieden sein können, eine geradkettige oder verzweigte aliphatische Gruppe mit 1 bis 30 Kohlenstoffatomen oder eine aromatische Gruppe wie Aryl oder Alkylaryl bedeuten. Die aliphatischen Gruppen können Heteroatome enthalten, insbesondere Sauerstoff, Stickstoff, Schwefel und Halogene. Die aliphatischen Gruppen sind beispielsweise unter den Gruppen Alkyl, Alkoxy, Polyoxyalkylen(C₂₋₆), Alkylamid, Alkyl(C₁₂₋₂₂)amidoalkyl(C₂₋₆), Alkyl(C₁₂₋₂₂)acetat und Hydroxyalkyl ausgewählt, die etwa 1 bis 30 Kohlenstoffatome aufweisen; X ist ein Anion, das unter den Halogeniden, Phosphaten, Acetaten, Lactaten, Alkyl(C₂₋₆)sulfaten, Alkyl- oder Alkylarylsulfonaten ausgewählt ist,
B) quartären Ammoniumalzen von hrnidazoliniurn, wie beispielsweise der folgenden Formel (V): 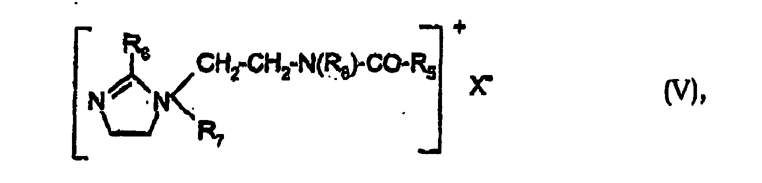 worin R₅ eine Alkenylgruppe oder Alkylgruppe mit 8 bis 30 Kohlenstoffatomen bedeutet, die beispielsweise von Talgfettsäuren abgeleitet sind, R₆ ein Wasserstoffatom, eine C₁₋₄-Algruppe oder eine Alkenyl- oder Alkylgruppe mit 8 bis 30 Kohlenstoffatomen ist, R₇ eine C₁₋₄-Alkylgruppe bedeutet, R₈ ein Wasserstoffatom oder eine C₁₋₄-Alkylgruppe ist und X ein Anion ist, das unter den Halogeniden, Phosphaten, Acetaten, Lactaten, Alkylsulfaten und Alkyl- oder Alkylaryl,sulfonaten ausgewählt ist.
C) quartären Diammoniumsalzen der Formel (VI): 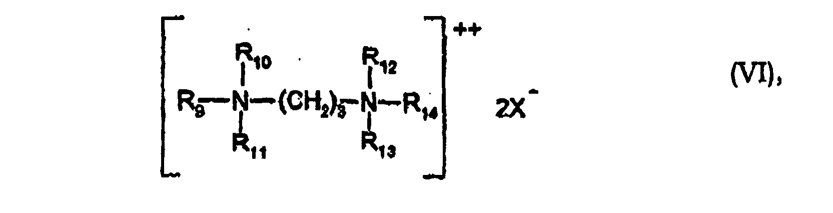 worin R₉ eine aliphatische Gruppe mit etwa 16 bis 30 Kohlenstoffatomen bedeutet, R₁₀, R₁₁, R₁₂, R₁₃ und R₁₄, die gleich oder verschieden sind, unter Wasserstoff oder einer Alkylgruppe mit 1 bis 4 Kohlenstoffatomen ausgewählt sind und X ein Anion ist, das unter den Halogeniden, Acetaten, Phosphaten, Nitraten und Methylsulfaten ausgewählt ist. Solche quartären Diammoniumsalze umfassen insbesondere das Propan(talg)diammoniumdichlorid.
D) quartäre Ammoniumsalze, die mindestens eine Esterfunktion der folgenden Formel (VII) enthalten: 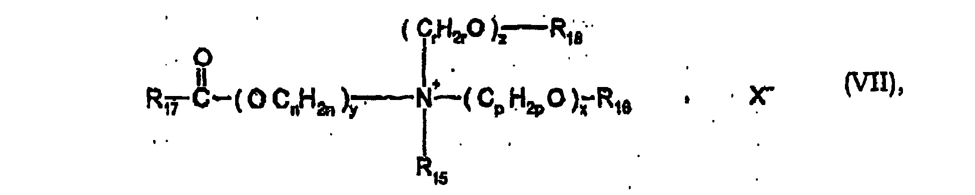 worin bedeuten:
- R₁₅ ist unter den C₁₋₆-Alkylgruppen und den Hydroxyalkyl- oder Dihydxoxyalkylgruppen mit 1 bis 6 Kohlenstoffatomen ausgewählt;
- R₁₆ ist ausgewählt unter:
- der Gruppe 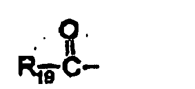
- geradkettigen oder verzweigten, gesättigten oder ungesättigten Kohlenwasserstoffgruppen R₂₀ mit 1 bis 22 Kohlenstoffatomen,
- dem Wasserstoffatom,
- Ris ist ausgewählt unter:
- der Gruppe 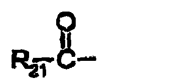
- geradkettigen oder verzweigten, gesättigten oder ungesättigten Kohlenwasserstoffgruppen R₂₂ mit 1 bis 6 Kohlenstoffatomen,
- dem Wasserstoffatom,
- R₁₇, R₁₉ und F₂₁, die gleich oder verschieden sind, sind unter den gesättigten oder ungesättigten, geradkettigen oder verzweigten Kohlenwasserstoffgruppen mit 7 bis 21 Kohlenstoffatomen ausgewählt;
- n, p und r, die gleich oder verschieden sind, sind ganze Zahlen von 2 bis 6;
- y ist eine ganze Zahl von 1 bis 10;
- x und z, die gleich oder verschieden sind, sind ganze Zahlen von 0 bis 10;
- X⁻ ist ein einfaches oder komplexes, organisches oder anorganisches Anion;
mit der Maßgabe, dass, wenn die Summe x + y + z im Bereich von 1 bis 15 liegt, R₁₆ R₂₀ bedeutet, wenn x 0 ist und R₁₈ R₂₂ bedeutet, wenn z 0 ist.

20. Nanoemulsion nach dem vorhergehenden Anspruch, **dadurch gekennzeichnet, dass** die kationischen grenzflächenaktiven Stoffe der Formel (IV) unter Tetraalkylammoniumchloriden, wie beispielsweise Dialkyldimethylammoniumchloriden oder Alkyltrimethylammoniumchloriden ausgewählt sind, worin die Alkylgruppe etwa 12 bis 22 Kohlenstoffatome aufweist, insbesondere Behenyltrimethylammoniumchlorid, Distearyldimethylammoniumchlorid, Cetyltrimethylammoniumchlorid, Benzyldimethylstearylammoniumchlorid oder auch Stearamidopropyldimethyl-(myristylacetat)ammoniumchlorid.

21. Nanoemulsion nach einem der Ansprüche 19 oder 20, **dadurch gekennzeichnet, dass** der kationische grenzflächenaktive Stoff unter den Behenyltrimethylammoniumsalzen, Stearamidopropyldimethyl(myristylacetat)ammoniumsalzen ausgewählt ist.

22. Nanoemulsion nach einem der Ansprüche 18 bis 21, **dadurch gekennzeichnet, dass** das zusätzliche kationische oder anionische amphiphile Lipid in den Nanoemulsionen in Konzentrationen von 0,01 bis 10 Gew.-%, bezogen auf das Gesamtgewicht der Nanoemulsion, und insbesondere 0,2 bis 5 Gew.-% enthalten ist.

23. Nanoemulsion nach einem der vorhergehenden Ansprüche, **dadurch gekennzeichnet, dass** das Öl unter den pflanzlichen Ölen, tierischen Ölen, synthetischen Ölen, Mineralölen, halogenierten Ölen, Estern einer anorganischen Säure und eines Alkohols, flüssigen Carbonsäureestern und Siliconen ausgewählt ist.

24. Nanoemulsion nach einem der Ansprüche 1 bis 23, **dadurch gekennzeichnet, dass** der Mengenanteil des Öls im Bereich von 2 bis 40 Gew.-% und vorzugsweise 4 bis 30 Gew.%, bezogen auf das Gesamtgewicht der Nanoemulsion, liegt.

25. Nanoemulsion nach einem der Ansprüche 1 bis 24, **dadurch gekennzeichnet, dass** sie einen wasserlöslichen oder fettlöslichen, kosmetischen oder dermopharmazeutischen Wirkstoff enthält.

26. Nanoemulsion nach einem der Ansprüche 1 bis 25, **dadurch gekennzeichnet, dass** sie eine Trübung von 60 bis 600 NTU besitzt.

27. Kosmetische Zusammensetzung zur topischen Anwendung, **dadurch gekennzeichnet, dass** sie aus einer Nanoemulsion nach einem der Ansprüche 1 bis 26 besteht oder eine Nanoemulsion nach einem der Ansprüche 1 bis 26 enthält.

28. Verwendung einer Nanoemulsion nach einem der Ansprüche 1 bis 26 als Produkt zur Pflege und/ oder zur Reinigung und/oder zum Schminken und/oder zum Abschminken der Haut des Körpers und/oder des Gesichts und/oder der Schleimhäute und/oder der Kopfhaut und/oder der Haare und/oder der Nägel und/oder der Wimpern und/oder der Augenbrauen oder in solchen Produkten.

29. Verfahren zur nicht-therapeutischen Behandlung der Haut, der Haare, der Schleimhäute, der Nägel, der Wimpern, der Augenbrauen und/oder der Kopfhaut, **dadurch gekennzeichnet, dass** auf die Haut, die Haare, die Schleimhäute, die Nägel, die Wimpern, die Augenbrauen oder die Kopfhaut eine Nanoemulsion nach einem der Ansprüche 1 bis 26 oder eine Zusammensetzung nach Anspruch 27 aufgetragen wird.

30. Verfahren zur Verdickung von Öl-in-Wasser-Nanoemulsionen, die Öltröpfchen mit einer zahlenmittleren Größe unter 150 nm enthält, wobei zu der Zusammensetzung ein kationisches Polymer gegeben wird, das mindestens eine hydrophobe Sequenz und mindestens eine hydrophile Sequenz aufweist.
